# FASCICULE DE BREVET EUROPEEN

(11) **EP 4 211 104 B1**
(45) Date de publication et mention de la délivrance du brevet: **03.07.2024**
(21) Numéro de dépôt: 21762585.4
(22) Date de dépôt: 06.09.2021
(51) Int. Cl.: C07C 37/62, C07C 39/27

(54) **PROCÉDÉ DE SYNTHÈSE DU 3,5-DIIODO-4-HYDROXY BENZYLALCOOL**
VERFAHREN ZUR HERSTELLUNG VON 3,5-DIIOD-4-HYDROXYBENZYLALKOHOL
PROCESS FOR THE SYNTHESIS OF 3,5-DIIODO-4-HYDROXYBENZYL ALCOHOL

(30) Priorité: 08.09.2020 FR 2009106
(43) Date de publication de la demande: 19.07.2023
(73) Titulaire: Innoverda, 94800 Villejuif (FR)
(72) Inventeur: ERDELMEIER, Irène, 75013 PARIS (FR); DAUNAY, Sylvain, 94170 Le Perreux sur Marne (FR)
(74) Mandataire: Marconnet, Sébastien
(86) Numéro de dépôt international: PCT/EP2021/074440
(87) Numéro de publication internationale: WO 2022/053418

(56) Documents cités:
- WO-A1-2020/137935
- WO-A2-2013/010102

## Description

L'invention concerne un nouveau procédé pour la synthèse du 4-hydroxy-3,5-diiodobenzyl alcool ou 4-hydroxy-3,5-diiodobenzenemethanol, CAS-N° 37987-26-1, de formule 1, 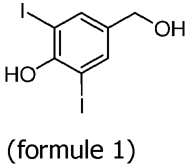 qui est un composé important pour l'étude du métabolisme de l'hormone L-thyroxine ainsi qu'un intermédiaire potentiel de synthèse notamment du Levothyrox.

### Etat de l'art

La synthèse décrite dans la littérature se déroule en deux étapes à partir du 4-hydroxybenzylaldehyde :

### 1. Iodination avec ICI :

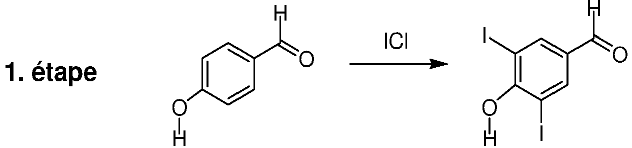

Décrit dans WO9522992A2 (page 28), WO2013010102A2 (page 98, 85%), Tetrahedr. Lett. 1997, 38 (40), 6965 et JACS 1959, 81, 871 (sans rendement) et aussi J. Chem. Soc. 1922, 121, 1055 (via la préparation d'un dérivé du mercure puis réaction avec KI/I2), et avec I2/HIO3 dans Chem. Ber. 1895, 2407.

### 2. Réduction en alcool :

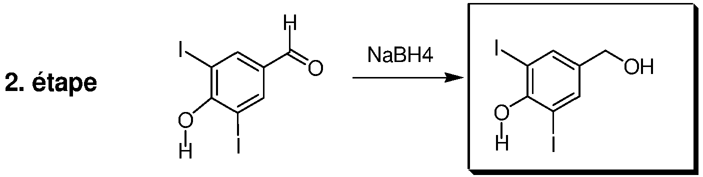

Décrit dans WO9522992A2 (page 30, avec BH3), WO2013010102A2 (page 99), Tetrahedr. Lett. 1997, 38 (40), 6965, et Chem. Eur. J. 2016, 22, 3105 (dans le Supporting Information Page 115, 99%), et Org. Lett. 2019, 21, 6504 et Monatsh. Chem. (1972), 103(4), 1178-93.

De son côté, WO2020/137935A1 décrit la préparation de l'alcool 4-hydroxy-3,5-diiodobenzyle en faisant réagir une solution méthanolique d'alcool 4-hydroxybenzyle avec du peroxyde d'hydrogène et de l'iode.

### BUTS DE L'INVENTION

L'invention a pour but principal de résoudre le problème technique de fournir un nouveau procédé de synthèse en une seule étape du composé de formule 1 ci-dessus.

L'invention a encore pour but principal de résoudre le problème technique de fournir un nouveau procédé de synthèse en une seule étape du composé de formule 1 ci-dessus avec d'excellents rendements. L'invention a encore pour but principal de résoudre le problème technique de fournir un nouveau procédé de synthèse en une seule étape du composé de formule 1 ci-dessus, à la température ambiante, avec d'excellents rendements et selon une solution simple peu coûteuse, utilisable à l'échelle industrielle.

### DESCRIPTION DETAILLEE DE L'INVENTION

L'invention résout pour la première fois l'ensemble des problèmes techniques énoncés ci-dessus, d'une manière simple, peu coûteuse, utilisable à l'échelle industrielle.

Ainsi, selon un premier aspect, la présente invention fournit un procédé de synthèse du 4-hydroxy-3,5-diiodo-benzylalcool, caractérisé en ce qu'il comprend la synthèse, en une seule étape, du 4-hydroxy-3,5-diiodo-benzylalcool de formule 1 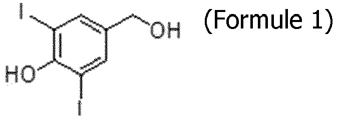 à partir du 4-hydroxybenzylalcool, en solution aqueuse à pH initial au moins égal à 7, contenant au moins 2 équivalents d'agent iodant, en particulier l'iode ou diiodure.

La iodination directe et la diiodination directe d'autres molécules du type phénol sont connus, mais d'une manière générale, il faut des conditions réactionnelles oxydantes, tel que présence de HIO3, H2O2, etc... voir notamment les références 9, 10.

L'invention réalise, d'une manière totalement surprenante pour un homme de l'art, une di-iodation directe du 4-hydroxybenzylalcool.

L'utilisation du 4-hydroxybenzylalcool est non évident pour un homme de l'art car l'état de la technique enseigne une iodination en présence d'un agent oxydant et l'homme de l'art devait s'attendre que la fonction benzylalcool soit oxydée dans ces conditions. En outre, l'homme de l'art devait s'attendre aussi qu'il soit difficile de s'arrêter au dérivé di-iodé, en raison du fait que l'on a un dérivé phénolique riche en électrons et donc très réactif par rapport à une substitution électrophile, ce qui est moins le cas pour le dérivé aldéhyde préconisé par l'homme de l'art depuis 1895, voir référence 11 et encore à partir de 1959, voir référence 4, soit depuis plus de 120 ans.

Il y avait donc un réel préjugé dans l'art à réaliser une diiodination directe du 4-hydroxybenzylalcool.

En outre, dans le cadre de l'invention, on obtient en une seule étape cette di-iodination avec des rendements excellents, c'est-à-dire d'au moins 75%, ce qui n'a jamais été obtenu auparavant, et ceci tout simplement avec un agent iodant, en particulier l'iode, sans ajouter un oxydant.

Selon une variante de réalisation du procédé de l'invention, la réaction a lieu à la température ambiante. Il est aussi surprenant selon l'invention, comme montré par les exemples qui suivent, que même à la température ambiante, la durée de réaction est courte de l'ordre d'environ 2H, en faisant un procédé excellent pour une production industrielle à faible coût.

Selon un mode de réalisation particulier, le procédé est caractérisé en ce que le pH initial est réglé à au moins 7 par ajout d'une base faible, en particulier choisie parmi le Disodiumhydrogène phosphate (Na2HPO4), KHCO3 ou le K2HPO4.

Selon un autre mode de réalisation particulier, le procédé est caractérisé en ce que le pH initial de la solution aqueuse est compris entre 7 et 11.

Selon encore un mode de réalisation particulier, le procédé est caractérisé en ce que le pH initial de la solution aqueuse est compris entre 7 et 9.

Selon un autre mode de réalisation particulier, le procédé est caractérisé en ce qu'on utilise au moins 2 équivalents de base faible, en particulier choisie parmi le Disodiumhydrogène phosphate (Na2HPO4), KHCO3 ou le K2HPO4.

Selon un autre mode de réalisation particulier, le procédé est caractérisé en ce que la solution aqueuse comprend au moins un alcool hydrosoluble.

Selon encore un autre mode de réalisation particulier, le procédé est caractérisé en ce que la proportion en poids en alcool hydrosoluble dans la solution aqueuse est comprise entre supérieure à zéro et environ 25%.

Selon une variante de réalisation particulière, la proportion en poids en alcool hydrosoluble dans la solution aqueuse est comprise entre 5 et 20%

Selon une autre variante de réalisation particulière, la proportion en poids en alcool hydrosoluble dans la solution aqueuse est comprise entre 5 et 15%.

Selon encore une variante de réalisation particulière, la proportion en poids en alcool hydrosoluble dans la solution aqueuse est d'environ 10%.

Selon encore un mode de réalisation particulier, le procédé est, caractérisé en ce que l'alcool hydrosoluble est un alcool inférieur en C1-C6, en particulier choisi parmi le méthanol, l'éthanol, le propanol, l'isopropanol, le butanol, le pentanol.

Selon un mode de réalisation particulier, le procédé est caractérisé en ce que la solution aqueuse comprend un mélange choisi parmi eau/ éthanol 9/1, eau/méthanol 9/1, eau/propanol ou isopropanol 9/1.

Selon un autre mode de réalisation particulier, le procédé est caractérisé en ce que la réaction a lieu en l'absence d'agent(s) oxydant(s).

Selon encore un autre mode de réalisation particulier, le procédé est caractérisé en ce que la solution aqueuse est monophasique.

Selon encore un autre mode de réalisation particulier, le procédé est caractérisé en ce qu'on réalise la réaction chimique suivante :

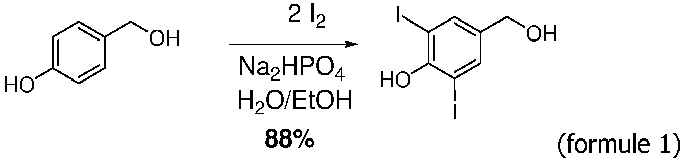

D'autres buts, caractéristiques et avantages de l'invention apparaîtront clairement à un Homme de l'art à la lumière des exemples de réalisation de l'invention donnés ci-après à titre d'illustration et qui ne sauraient en aucune façon limiter la portée de l'invention définie par les revendications. Dans la description incluant les exemples et les revendications, la pression est la pression atmosphérique, la température est donnée en degrés Celsius, les pourcentages sont donnés en poids, sauf indications contraires. La température ambiante est la température habituelle de la pièce, incluant une pièce climatisée ; elle est comprise par l'homme de l'art comme étant généralement comprise entre 18 °C et 25°C.

### PARTIE EXPERIMENTALE

### I- EXEMPLES SELON L'INVENTION

### Exemple 1 selon l'invention : Protocole optimal

12,41 g (100 mmoles) de 4-hydroxybenzyl alcool, 39,95 g (220 mmoles) de di-sodium hydrogénophosphate et 56,4 g (220 mmoles) d'iode sont mis en solution dans 100 ml d'éthanol absolu et 900 ml d'eau déminéralisée. Le pH initial de la solution est 8. Sous forte agitation, la solution est agitée à température ambiante pendant au moins 2 heures. 8,8 ml (100 mmoles) d'acide chlorhydrique concentrée est ajouté au milieu réactionnel pour obtenir un pH de 3. La suspension obtenue est filtrée et le solide obtenu lavé par 50 ml d'une solution de thiosulfate à 5% et 3 fois par 50 ml d'eau. 32,8g **(88%)** de 4-hydroxy-3,5-diiodobenzyl alcool sous forme d'une poudre sont obtenus après séchage. Le spectre 1H-NMR est conforme avec la structure et la littérature [ref 5]

1H-NMR (400 MHz, DMSO-d6) δ 9.40 (s, 1H), 7.67 (s, 2H), 5.20 (t, J = 5.5 Hz, 1H), 4.35 (d, J = 5.5 Hz, 2H).

### Exemple 2 selon l'invention : Changement de la base ajoutée : KHCO3

1,24 g (10 mmoles) de 4-hydroxybenzyl alcool, 2,22 g (22 mmoles) de potassium hydrogénocarbonate et 5,64 g (22 mmoles) d'iode sont mis en solution dans 10 ml d'éthanol absolu et 90 ml d'eau déminéralisée. Le pH initial de la solution est 8,7. Sous forte agitation, la solution est agitée à température ambiante pendant au moins 2 heures. Après dilution par ajout de 100 ml d'eau, la suspension obtenue est filtrée et le solide obtenu lavé par 20 ml d'une solution de thiosulfate à 5% et 3 fois avec 20 ml d'eau. 3,20g **(85%)** de 4-hydroxy-3,5-diiodobenzyl alcool sous forme d'une poudre sont obtenus après séchage.

### Exemple 3 selon l'invention : Changement de la base ajoutée : K2HPO4

1,24 g (10 mmoles) de 4-hydroxybenzyl alcool, 3,83 g (22 mmoles) de di-potassium hydrogénophosphate et 5,64 g (22 mmoles) d'iode sont mis en solution dans 10 ml d'éthanol absolu et 90 ml d'eau déminéralisée. Le pH initial de la solution est 7,5. Sous forte agitation, la solution est agitée à température ambiante pendant au moins 2 heures. Après dilution par ajout de 100 ml d'eau, la suspension obtenue est filtrée et le solide obtenu lavé par 20 ml d'une solution de thiosulfate à 5% et 3 fois avec 20 ml d'eau. 3,12g **(83%)** de 4-hydroxy-3,5-diiodobenzyl alcool sous forme d'une poudre est obtenue après séchage.

### Exemple 4 selon l'invention : Modification de l'alcool hydrosoluble : Méthanol au lieu d'éthanol

En procédant comme décrit dans l'exemple 1, en utilisant une solution aqueuse de méthanol et d'eau dans un rapport 10/90, le 4-hydroxy-3,5-diiodobenzyl alcool est obtenu sous forme d'une poudre après séchage avec un rendement de 85%.

### Exemple 5 selon l'invention: Modification de l'alcool hydrosoluble: Isopropanol au lieu d'éthanol

En procédant comme décrit dans l'exemple 1, en utilisant une solution aqueuse d'isopropanol et d'eau dans un rapport 10/90, le 4-hydroxy-3,5-diiodobenzyl alcool est obtenu sous forme d'une poudre après séchage avec un rendement de **87%.**

### Exemple 6 selon l'invention : Modification du milieu en utilisant une solution aqueuse sans alcool hydrosoluble

En procédant comme décrit dans l'exemple 1, en utilisant une solution aqueuse sans alcool, le 4-hydroxy-3,5-diiodobenzyl alcool est obtenu sous forme d'une poudre après séchage avec un rendement de **88%,** contenant environ 5% du sous-produit mono-iodé.

### Exemple 7 selon l'invention : Changement de rapport eau/alcool à 95/5 au lieu de 90/10

En procédant comme décrit dans l'exemple 1, en utilisant une solution aqueuse d'éthanol et d'eau dans un rapport 5 :95, le 4-hydroxy-3,5-diiodobenzyl alcool est obtenu sous forme d'une poudre après séchage avec un rendement de **82%.**

### Exemple 8 selon l'invention : Changement de rapport eau/alcool à 80/20 au lieu de 90/10

En procédant comme décrit dans l'exemple 1, en utilisant une solution aqueuse d'éthanol et d'eau dans un rapport 20 : 80, le 4-Hydroxy-3,5-diiodobenzyl alcool est obtenu sous forme d'une poudre après séchage avec un rendement de **78%.**

### II- EXEMPLES COMPARATIFS

### Exemple 9 COMPARATIF 1: Changement de la base ajoutée : acétate de sodium

1,24 g (10 mmoles) de 4-hydroxybenzyl alcool, 1,82 g (22 mmoles) de sodium acétate et 5,13 g (20 mmoles) diiode sont mis en solution dans 10 ml d'éthanol absolu et 90 ml d'eau déminéralisée. Le pH initial de la solution est 6. Sous forte agitation, la solution est agitée à température ambiante pendant au moins 2 heures. La suspension obtenue est filtrée et le solide obtenu lavé par 20 ml d'une solution de thiosulfate à 5% et 3 fois par 20 ml d'eau. 2,03g **(53%)** de 4-hydroxy-3,5-diiodobenzyl alcool sous forme d'une poudre sont obtenus après séchage.

### Exemple 10 COMPARATIF 2 : Changement de la base ajoutée : Sodium dihydrogène phosphate

En procédant comme décrit dans l'exemple 1, mais en utilisant 2,2 equiv. de NaH2PO4 au lieu de 2,2 equiv de Na2HPO4, le pH initial de la solution est 4,5. Le produit désiré est formé mais très minoritaire (< **30%),** et ne peut pas être isolé par précipitation.

### Exemple 11 COMPARATIF 3 : Changement de la base ajoutée : Potassium dihydrogène phosphate

En procédant comme décrit dans l'exemple 1, mais en utilisant 2,2 equiv. de KH2PO4 au lieu de 2,2 equiv de Na2HPO4, le pH initial de la solution est 4. Le produit désiré est formé en trace (< **15%),** et ne peut pas être isolé par précipitation.

### Exemple 12 COMPARATIF 4 : Changement d'équivalent de I2

1,24 g (10 mmoles) de 4-hydroxybenzyl alcool, 3,83 g (22 mmoles) de di-sodium hydrogénophosphate et 3,58 g (14 mmoles) diiode sont mis en solution dans 10 ml d'éthanol absolu et 90 ml d'eau déminéralisée. Le pH initial est 8,5. Sous forte agitation, la solution est agitée à température ambiante pendant 20 heures. 100 ml d'eau est ajouté au milieu réactionnel puis la suspension obtenue est récupérée par filtration et lavée par 20 ml d'une solution de thiosulfate à 5% et 3 fois 20 ml d'eau. 2,06 g (**55%**) de 4-hydroxy-3,5-diiodobenzyl alcool sous forme d'une poudre est obtenue après séchage.

Il est observé que le temps de réaction de 20H nécessaire en comparaison avec 2 heures dans le cadre de l'invention, est incompatible avec une production industrielle à faible coût.

### Exemple 13 COMPARATIF 5 : Changement de la température réactionnelle : 60°C au lieu de la température ambiante

En procédant comme décrit dans l'exemple 1, mais en chauffant le milieu à 60°C pendant une heure, le 4-hydroxy-3,5-diiodobenzyl alcool est formé (environ **50%**), avec des produits de dégradation.

### Exemple 14 COMPARATIF 6 : Changement de rapport eau/éthanol à 70/30 au lieu de 90/10

1,24 g (10 mmoles) de 4-hydroxybenzyl alcool, 3,83 g (22 mmoles) de di-sodium hydrogénophosphate et 5,12 g (20 mmoles) diiode sont mis en solution dans 30 ml d'éthanol absolu et 70 ml d'eau déminéralisée. Le pH initial est 8,5. Sous forte agitation, la solution est agitée à température ambiante pendant 20 heures. 100 ml d'eau est ajouté au milieu réactionnel puis la suspension obtenue est filtrée et le solide obtenu lavée par 20 ml d'une solution de thiosulfate à 5% et 3 fois 20 ml d'eau. 2,13 g (**57%**) de 4-hydroxy-3,5-diiodobenzyl alcool sous forme d'une poudre est obtenue après séchage.

### Exemple 15 COMPARATIF 7 : Changement de rapport eau/éthanol à 50/50 au lieu de 90/10

En procédant comme décrit dans l'exemple 1, en utilisant une solution aqueuse d'éthanol et d'eau dans un rapport 50/50, 4-hydroxy-3,5-diiodobenzyl alcool est présent en faible rendement (< **20%).** On détecte majoritairement des produits de dégradation.

### Exemple 16 COMPARATIF 8 : Réaction dans une solution éthanolique non-aqueuse

1,24 g (10 mmoles) de 4-hydroxybenzyl alcool, 2,27 g (22 mmoles) triéthylamine et 5,64 g (22 mmoles) diiode sont mis en solution dans 100 ml d'éthanol absolu. Le pH initial est 8,5. Sous forte agitation, la solution est agitée à température ambiante pendant 2 heures. Le 4-hydroxy-3,5-diiodobenzyl alcool est présent en faible rendement (< **25%).** On détecte majoritairement des produits de dégradation.

### Références

1. Blaney, Jeffrey M.; Cohen, Fred; PCT Int. Appl. (1995), WO 9522992 A2.
2. Latham, Keith R; PCT Int. Appl. (2013), WO 2013010102 A2.
3. Salamonczyk, Grzegorz M.; Oza, Vibha B.; Sih, Charles J.; Tetrahedr. Lett. 1997, 38 (40), 6965.
4. Teruo Matsuura and H. J. Cahnmann; JACS 1959, 81, 871.
5. Etienne André, Baptiste Boutonnet, Pauline Charles, Cyril Martini, Juan-Manuel Aguiar-Hualde, Sylvain Latil, Vincent Gurineau, Karim Hammad, Priyanka Ray, Régis Guillot and Vincent Huc; Chem. Eur. J. 2016, 22, 3105.
6. Michael F. McLaughlin, Elisabetta Massolo, Thomas A. Cope, and Jeffrey S. Johnson; Org. Lett. 2019, 21, 6504.
7. P. Claus, P. Schilling, J. S. Gratzl, K. Kratzl; Monatsh. Chem. (1972), 103(4), 1178.
8. T. A. Henry, T. M. Sharp; J. Chem. Soc., Trans. 1922, 121, 1055.
9. Citterio, A.; Battistini, E.; Belnome, D.; Buonsanti, F.; Lattuada, L.; Leonardi, G.; Uggeri, F.; Vignale, E.; Visigalli, M. Process for the iodination of phenolic derivatives; US Patent 8,766,003, Jul 1, 2014.
10. Gallo, R. D. C.; Gebara, K. S.; Muzzi, R. M.; Raminelli, C. Efficient and Selective Iodination of Phenols Promoted by Iodine and Hydrogen Peroxide in Water. Journal of the Brazilian Chemical Society 2010, 21 (4), 770-774. http://dx.doi.ora/10.1590/S0103-50532010000400026
11. C. Paal, Chem. Ber. 1895, 28, 2407-2414.

## Revendications

1. Procédé de synthèse du 4-hydroxy-3,5-diiodobenzyl alcool, **caractérisé en ce qu'**il comprend la synthèse, en une seule étape, du 4-hydroxy- 3,5-diiodo-benzylalcool de formule 1 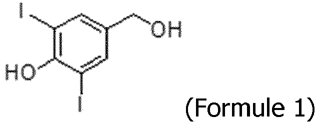 à partir du 4-hydroxybenzylalcool, en solution aqueuse à pH initial au moins égal à 7, contenant au moins 2 équivalents d'agent iodant, en particulier iode ou diiodure.

2. Procédé selon la revendication 1, **caractérisé en ce que** le pH initial est réglé à au moins 7 par ajout d'une base faible, en particulier choisie parmi le Disodiumhydrogène phosphate (Na2HPO4), KHCO3 ou le K2HPO4.

3. Procédé selon la revendication 1 ou 2, **caractérisé en ce que** le pH initial de la solution aqueuse est compris entre 7 et 11.

4. Procédé selon une des revendications 1, 2 ou 3, **caractérisé en ce que** le pH initial de la solution aqueuse est compris entre 7 et 9.

5. Procédé selon l'une des revendications 1 à 4, **caractérisé en ce qu'**on utilise au moins 2 équivalents de base faible, en particulier choisie parmi le Disodiumhydrogène phosphate (Na2HPO4), KHCO3 ou le K2HPO4.

6. Procédé selon l'une des revendications 1 à 5, **caractérisé en ce que** la solution aqueuse comprend au moins un alcool hydrosoluble, en particulier à une proportion en poids supérieure à zéro et 25%.

7. Procédé selon l'une des revendications 1 à 6, **caractérisé en ce que** la solution aqueuse comprend au moins un alcool hydrosoluble, à une proportion en poids comprise entre 5 et 20%, en particulier comprise entre 5 et 15%.

8. Procédé selon la revendication 6 ou 7, **caractérisé en ce que** l'alcool hydrosoluble est un alcool inférieur en C1-C6, en particulier choisi parmi le méthanol, l'éthanol, le propanol, l'isopropanol, le butanol, le pentanol.

9. Procédé selon l'une des revendications 1 à 8, **caractérisé en ce que** la solution aqueuse comprend un mélange choisi parmi eau/ éthanol 9/1, eau/méthanol 9/1, eau/propanol ou isopropanol 9/1.

10. Procédé selon l'une des revendications 1 à 9, **caractérisé en ce que** la réaction a lieu en l'absence d'agent(s) oxydant(s).

11. Procédé selon l'une des revendications 1 à 10, **caractérisé en ce que** la réaction a lieu à la température ambiante.

12. Procédé selon l'une des revendications 1 à 11, **caractérisé en ce qu'**on réalise la réaction chimique suivante :

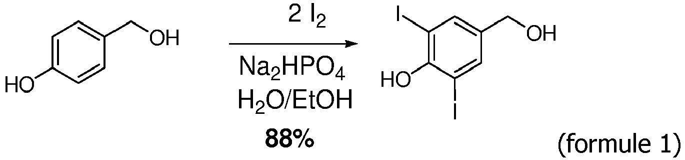

## Patentansprüche

1. Verfahren zur Synthese von 4-Hydroxy-3,5-diiodobenzyl Alkohol, **dadurch gekennzeichnet, dass** die Synthese der Verbindung der Formel 1, 4-Hydroxy-3,5-diiodo-benzyl Alkohol, 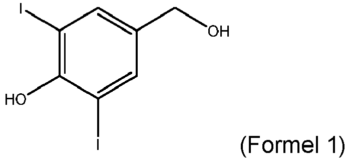 in nur einem Schritt durch Umsetzung von 4-Hydroxybenzylalcohol in wässriger Lösung mit mindestens 2 Äquivalenten eines iodierenden Reagenzes, vorzugsweise lod, bei einem anfänglichen pH-Wert von mindestens 7 erfolgt.

2. Verfahren nach Anspruch 1, **dadurch gekennzeichnet, dass** der anfängliche pH-Wert auf mindestens 7 durch Zugabe einer schwachen Base, ausgewählt insbesondere aus Dinatriumhydrogenphosphat (Na2HPO4), Kaliumhydrogencarbonat (KHCO3), oder Dikaliumhydrogenphosphat (K2HPO4) eingestellt wird.

3. Verfahren nach Anspruch 1 oder 2, **dadurch gekennzeichnet, dass** der anfängliche pH-Wert zwischen 7 und 11 eingestellt wird.

4. Verfahren nach einem der Ansprüche 1 bis 3, **dadurch gekennzeichnet, dass** der anfängliche pH-Wert zwischen 7 und 9 eingestellt wird.

5. Verfahren nach einem der Ansprüche 1 bis 4, **dadurch gekennzeichnet, dass** mindestens 2 Äquivalente einer schwachen Base verwendet werden, die insbesondere ausgewählt sind aus Dinatriumhydrogenphosphat (Na2HPO4), Kaliumhydrogencarbonat (KHCO3), oder Dikaliumhydrogenphosphat (K2HPO4).

6. Verfahren nach einem der Ansprüche 1 bis 5, **gekennzeichnet dadurch, dass** die wässrige Lösung mindestens einen wasserlöslichen Alkohol in Gewichtsprozenten zwischen mehr als 0% bis 25% enthält.

7. Verfahren nach einem der Ansprüche 1 bis 6, **gekennzeichnet dadurch, dass** die wässrige Lösung mindestens einen wasserlöslichen Alkohol in Gewichtsprozenten zwischen 5 und 20%, vorteilshafterweise zwischen 5 und 15%, enthält.

8. Verfahren nach einem der Ansprüche 6 oder 7, **gekennzeichnet dadurch, dass** der wasserlösliche Alkohol ein niedriger Alkohol mit 1 bis 6 Kohlenstoffatomen ist, insbesondere ausgewählt aus Methanol, Äthanol, Propanol, Isopropanol, Butanol oder Pentanol.

9. Verfahren nach einem der Ansprüche 1 bis 8, **gekennzeichnet dadurch, dass** die wässrige Lösung eine Mischung ausgewählt aus Wasser/Äthanol 9/1, Wasser/Methanol 9/1, Wasser/Propanol 9/1 oder Wasser/Isopropanol 9/1 ist.

10. Verfahren entsprechend der Ansprüche 1 bis 9, **gekennzeichnet dadurch, dass** die Reaktion ohne den Zusatz oxidierender Reagenzien stattfindet.

11. Verfahren entsprechend der Ansprüche 1 bis 10, **gekennzeichnet dadurch, dass** die Reaktion bei Raumtemperatur stattfindet.

12. Verfahren entsprechend der Ansprüche 1 bis 11, **gekennzeichnet dadurch, dass** die folgende chemische Reaktion ausgeführt wird:

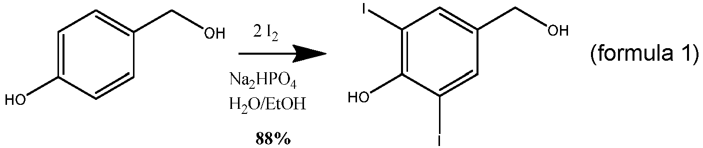

## Claims

1. Method for synthesizing 4-hydroxy-3,5-diiodobenzyl alcohol, **characterized in that** it comprises the synthesis, in just one step, of 4-hydroxy-3,5-diiodo-benzyl alcohol of formula 1 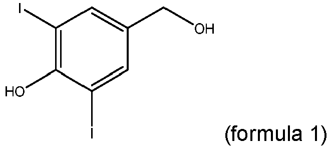 from 4-hydroxybenzylalcohol, in aqueous solution having an initial pH of at least 7, containing at least 2 equivalents of iodizing agent, in particular iodine or diiodide.

2. Method according to claim 1, **characterized in that** the initial pH is set to at least 7, by addition of a weak base, in particular selected from disodium hydrogen phosphate (Na2HPO4), KHCO3, or K2HPO4.

3. Method according to either claim 1 or claim 2, **characterized in that** the initial pH of the aqueous solution is between 7 and 11.

4. Method according to any of claims 1, 2 or 3, **characterized in that** the initial pH of the aqueous solution is between 7 and 9.

5. Method according to any of claims 1 to 4, **characterized in that** at least 2 equivalents of weak base are used, said base in particular being selected from disodium hydrogen phosphate (Na2HPO4), KHCO3, or K2HPO4.

6. Method according to any of claims 1 to 5, **characterized in that** the aqueous solution comprises at least one hydrosoluble alcohol, in particular at a proportion by weight of between more than zero and 25%.

7. Method according to any of claims 1 to 6, **characterized in that** the aqueous solution comprises at least one hydrosoluble alcohol, at a proportion by weight of between 5 and 20%, in particular between 5 and 15%.

8. Method according to either claim 6 or claim 7, **characterized in that** the hydrosoluble alcohol is a lower alcohol in C1-C6, in particular selected from methanol, ethanol, propanol, isopropanol, butanol, pentanol.

9. Method according to any of claims 1 to 8, **characterized in that** the aqueous solution comprises a mixture selected from water/ethanol 9/1, water/methanol 9/1, water/propanol or isopropanol 9/1.

10. Method according to any of claims 1 to 9, **characterized in that** the reaction takes place in the absence of oxidizing agent(s).

11. Method according to any of claims 1 to 10, **characterized in that** the reaction takes place at ambient temperature.

12. Method according to any of claims 1 to 11, **characterized in that** the following chemical reaction is carried out:
